# EUROPEAN PATENT APPLICATION

(11) **EP 4 008 338 A1**
(43) Date of publication of application: **08.06.2022**
(21) Application number: 20934983.6
(22) Date of filing: 16.12.2020
(51) Int. Cl.: A61K 38/18, A61P 1/00, A61P 35/00

(54) **USE OF FGF21 IN PREPARATION OF MEDICINE FOR TREATING COLORECTAL CANCER**

(30) Priority: 12.05.2020 CN 202010396043
(71) Applicant: Jiangnan University, Wuxi, Jiangsu 214122 (CN)
(72) Inventor: ZHU, Shenglong, Wuxi, Jiangsu 214122 (CN); CHEN, Yongquan, Wuxi, Jiangsu 214122 (CN); WANG, Zhen., Wuxi, Jiangsu 214122 (CN)
(74) Representative: Ipside
(86) International application number: PCT/CN2020/136762
(87) International publication number: WO 2021/227493

(57) **Abstract**

The present invention discloses application of FGF21 in the preparation of medicines for treating colorectal cancer, and belongs to the technical field of medicine. The FGF21 protein provided by the present invention is a secreted protein composed of about 210 amino acids. The present invention found that FGF21 has the effect of treating colorectal tumors for the first time. It is proved by cytology and animal experiments that FGF21 can inhibit the proliferation, invasion and migration of colorectal tumor cell lines; can significantly reduce the size of tumor lesions in mouse models. The medicines prepared from FGF21 as the active ingredient for treating colorectal cancer has good safety and long duration of drug action, while it protects organs from damage and has the effect of improving lipid metabolism disorders.

SEQUENCE LISTING

<110>  Jiangnan University

<120>  Application of FGF21 in preparation of medicine for treating
       colorectal cancer

<130>  YGHY-2021-56

<150>  PCT/CN2020/136762
<151>  2020-12-16

<160>  14

<170>  PatentIn version 3.3

<210>  1
<211>  178
<212>  PRT
<213>  Artificial Sequence

<220>
<223>  Protein translated from synthetic DNA

<400>  1

Ala Asp Ser Ser Pro Leu Leu Gln Phe Gly Gly Gln Val Arg Gln Arg
1               5                   10                  15

Tyr Leu Tyr Thr Asp Asp Ala Gln Arg Thr Glu Ala His Leu Glu Ile
            20                  25                  30

Arg Glu Asp Gly Thr Val Gly Gly Ala Ala Asp Gln Ser Pro Glu Ser
        35                  40                  45

Leu Leu Gln Leu Lys Ala Leu Lys Pro Gly Val Ile Gln Ile Leu Gly
    50                  55                  60

Val Arg Thr Pro Arg Phe Leu Cys Gln Arg Pro Asp Gly Ala Leu Tyr
65                  70                  75                  80

Gly Ser Leu His Phe Asp Pro Glu Ala Cys Ser Phe Arg Glu Leu Leu
                85                  90                  95

Leu Glu Asp Gly Tyr Asn Val Tyr Gln Ser Glu Ala His Gly Leu Pro
            100                 105                 110

Leu His Leu Pro Gly Asn Lys Ser Pro His Arg Asp Pro Ala Pro Arg
        115                 120                 125

Gly Pro Ala Arg Phe Leu Pro Leu Pro Phe Leu Pro Pro Ala Leu Pro
    130                 135                 140

Glu Pro Pro Gly Ile Leu Gly Pro Gln Pro Pro Asp Val Gly Ser Ser

145                 150                 155                 160

Asp Pro Leu Ser Met Val Gly Pro Ser Gln Gly Arg Ser Pro Ser Tyr
                165                 170                 175

Ala Ser

<210>  2
<211>  784
<212>  PRT
<213>  Artificial Sequence

<220>
<223>  Protein translated from synthetic DNA

<400>  2

Ala Asp Ser Ser Pro Leu Leu Gln Phe Gly Gly Gln Val Arg Gln Arg
1               5                   10                  15

Tyr Leu Tyr Thr Asp Asp Ala Gln Arg Thr Glu Ala His Leu Glu Ile
            20                  25                  30

Arg Glu Asp Gly Thr Val Gly Gly Ala Ala Asp Gln Ser Pro Glu Ser
        35                  40                  45

Leu Leu Gln Leu Lys Ala Leu Lys Pro Gly Val Ile Gln Ile Leu Gly
    50                  55                  60

Val Arg Thr Pro Arg Phe Leu Cys Gln Arg Pro Asp Gly Ala Leu Tyr
65                  70                  75                  80

Gly Ser Leu His Phe Asp Pro Glu Ala Cys Ser Phe Arg Glu Leu Leu
                85                  90                  95

Leu Glu Asp Gly Tyr Asn Val Tyr Gln Ser Glu Ala His Gly Leu Pro
            100                 105                 110

Leu His Leu Pro Gly Asn Lys Ser Pro His Arg Asp Pro Ala Pro Arg
        115                 120                 125

Gly Pro Ala Arg Phe Leu Pro Leu Pro Phe Leu Pro Pro Ala Leu Pro
    130                 135                 140

Glu Pro Pro Gly Ile Leu Gly Pro Gln Pro Pro Asp Val Gly Ser Ser
145                 150                 155                 160

Asp Pro Leu Ser Met Val Gly Pro Ser Gln Gly Arg Ser Pro Ser Tyr
                165                 170                 175

Ala Ser Gly Gly Gly Gly Ser Gly Gly Gly Gly Ser Gly Gly Gly Gly
            180                 185                 190

Ser Arg Gly Val Phe Arg Arg Asp Ala His Lys Ser Glu Val Ala His
        195                 200                 205

Arg Phe Lys Asp Leu Gly Glu Glu Asn Phe Lys Ala Leu Val Leu Ile
    210                 215                 220

Ala Phe Ala Gln Tyr Leu Gln Gln Cys Pro Phe Glu Asp His Val Lys
225                 230                 235                 240

Leu Val Asn Glu Val Thr Glu Phe Ala Lys Thr Cys Val Ala Asp Glu
                245                 250                 255

Ser Ala Glu Asn Cys Asp Lys Ser Leu His Thr Leu Phe Gly Asp Lys
            260                 265                 270

Leu Cys Thr Val Ala Thr Leu Arg Glu Thr Tyr Gly Glu Met Ala Asp
        275                 280                 285

Cys Cys Ala Lys Gln Glu Pro Glu Arg Asn Glu Cys Phe Leu Gln His
    290                 295                 300

Lys Asp Asp Asn Pro Asn Leu Pro Arg Leu Val Arg Pro Glu Val Asp
305                 310                 315                 320

Val Met Cys Thr Ala Phe His Asp Asn Glu Glu Thr Phe Leu Lys Lys
                325                 330                 335

Tyr Leu Tyr Glu Ile Ala Arg Arg His Pro Tyr Phe Tyr Ala Pro Glu
            340                 345                 350

Leu Leu Phe Phe Ala Lys Arg Tyr Lys Ala Ala Phe Thr Glu Cys Cys
        355                 360                 365

Gln Ala Ala Asp Lys Ala Ala Cys Leu Leu Pro Lys Leu Asp Glu Leu
    370                 375                 380

Arg Asp Glu Gly Lys Ala Ser Ser Ala Lys Gln Arg Leu Lys Cys Ala
385                 390                 395                 400

Ser Leu Gln Lys Phe Gly Glu Arg Ala Phe Lys Ala Trp Ala Val Ala
                405                 410                 415

Arg Leu Ser Gln Arg Phe Pro Lys Ala Glu Phe Ala Glu Val Ser Lys

            420                 425                 430

Leu Val Thr Asp Leu Thr Lys Val His Thr Glu Cys Cys His Gly Asp
        435                 440                 445

Leu Leu Glu Cys Ala Asp Asp Arg Ala Asp Leu Ala Lys Tyr Ile Cys
    450                 455                 460

Glu Asn Gln Asp Ser Ile Ser Ser Lys Leu Lys Glu Cys Cys Glu Lys
465                 470                 475                 480

Pro Leu Leu Glu Lys Ser His Cys Ile Ala Glu Val Glu Asn Asp Glu
                485                 490                 495

Met Pro Ala Asp Leu Pro Ser Leu Ala Ala Asp Phe Val Glu Ser Lys
            500                 505                 510

Asp Val Cys Lys Asn Tyr Ala Glu Ala Lys Asp Val Phe Leu Gly Met
        515                 520                 525

Phe Leu Tyr Glu Tyr Ala Arg Arg His Pro Asp Tyr Ser Val Val Leu
    530                 535                 540

Leu Leu Arg Leu Ala Lys Thr Tyr Glu Thr Thr Leu Glu Lys Cys Cys
545                 550                 555                 560

Ala Ala Ala Asp Pro His Glu Cys Tyr Ala Lys Val Phe Asp Glu Phe
                565                 570                 575

Lys Pro Leu Val Glu Glu Pro Gln Asn Leu Ile Lys Gln Asn Cys Glu
            580                 585                 590

Leu Phe Glu Gln Leu Gly Glu Tyr Lys Phe Gln Asn Ala Leu Leu Val
        595                 600                 605

Arg Tyr Thr Lys Lys Val Pro Gln Val Ser Thr Pro Thr Leu Val Glu
    610                 615                 620

Val Ser Arg Asn Leu Gly Lys Val Gly Ser Lys Cys Cys Lys His Pro
625                 630                 635                 640

Glu Ala Lys Arg Met Pro Cys Ala Glu Asp Tyr Leu Ser Val Val Leu
                645                 650                 655

Asn Gln Leu Cys Val Leu His Glu Lys Thr Pro Val Ser Asp Arg Val
            660                 665                 670

Thr Lys Cys Cys Thr Glu Ser Leu Val Asn Arg Arg Pro Cys Phe Ser
        675                 680                 685

Ala Leu Glu Val Asp Glu Thr Tyr Val Pro Lys Glu Phe Asn Ala Glu
    690                 695                 700

Thr Phe Thr Phe His Ala Asp Ile Cys Thr Leu Ser Glu Lys Glu Arg
705                 710                 715                 720

Gln Ile Lys Lys Gln Thr Ala Leu Val Glu Leu Val Lys His Lys Pro
                725                 730                 735

Lys Ala Thr Lys Glu Gln Leu Lys Ala Val Met Asp Asp Phe Ala Ala
            740                 745                 750

Phe Val Glu Lys Cys Cys Lys Ala Asp Asp Lys Glu Thr Cys Phe Ala
        755                 760                 765

Glu Glu Gly Lys Lys Leu Val Ala Ala Ser Gln Ala Ala Leu Gly Leu
    770                 775                 780

<210>  3
<211>  784
<212>  PRT
<213>  Artificial Sequence

<220>
<223>  Protein translated from synthetic DNA

<400>  3

Arg Gly Val Phe Arg Arg Asp Ala His Lys Ser Glu Val Ala His Arg
1               5                   10                  15

Phe Lys Asp Leu Gly Glu Glu Asn Phe Lys Ala Leu Val Leu Ile Ala
            20                  25                  30

Phe Ala Gln Tyr Leu Gln Gln Cys Pro Phe Glu Asp His Val Lys Leu
        35                  40                  45

Val Asn Glu Val Thr Glu Phe Ala Lys Thr Cys Val Ala Asp Glu Ser
    50                  55                  60

Ala Glu Asn Cys Asp Lys Ser Leu His Thr Leu Phe Gly Asp Lys Leu
65                  70                  75                  80

Cys Thr Val Ala Thr Leu Arg Glu Thr Tyr Gly Glu Met Ala Asp Cys
                85                  90                  95

Cys Ala Lys Gln Glu Pro Glu Arg Asn Glu Cys Phe Leu Gln His Lys

            100                 105                 110

Asp Asp Asn Pro Asn Leu Pro Arg Leu Val Arg Pro Glu Val Asp Val
        115                 120                 125

Met Cys Thr Ala Phe His Asp Asn Glu Glu Thr Phe Leu Lys Lys Tyr
    130                 135                 140

Leu Tyr Glu Ile Ala Arg Arg His Pro Tyr Phe Tyr Ala Pro Glu Leu
145                 150                 155                 160

Leu Phe Phe Ala Lys Arg Tyr Lys Ala Ala Phe Thr Glu Cys Cys Gln
                165                 170                 175

Ala Ala Asp Lys Ala Ala Cys Leu Leu Pro Lys Leu Asp Glu Leu Arg
            180                 185                 190

Asp Glu Gly Lys Ala Ser Ser Ala Lys Gln Arg Leu Lys Cys Ala Ser
        195                 200                 205

Leu Gln Lys Phe Gly Glu Arg Ala Phe Lys Ala Trp Ala Val Ala Arg
    210                 215                 220

Leu Ser Gln Arg Phe Pro Lys Ala Glu Phe Ala Glu Val Ser Lys Leu
225                 230                 235                 240

Val Thr Asp Leu Thr Lys Val His Thr Glu Cys Cys His Gly Asp Leu
                245                 250                 255

Leu Glu Cys Ala Asp Asp Arg Ala Asp Leu Ala Lys Tyr Ile Cys Glu
            260                 265                 270

Asn Gln Asp Ser Ile Ser Ser Lys Leu Lys Glu Cys Cys Glu Lys Pro
        275                 280                 285

Leu Leu Glu Lys Ser His Cys Ile Ala Glu Val Glu Asn Asp Glu Met
    290                 295                 300

Pro Ala Asp Leu Pro Ser Leu Ala Ala Asp Phe Val Glu Ser Lys Asp
305                 310                 315                 320

Val Cys Lys Asn Tyr Ala Glu Ala Lys Asp Val Phe Leu Gly Met Phe
                325                 330                 335

Leu Tyr Glu Tyr Ala Arg Arg His Pro Asp Tyr Ser Val Val Leu Leu
            340                 345                 350

Leu Arg Leu Ala Lys Thr Tyr Glu Thr Thr Leu Glu Lys Cys Cys Ala
        355                 360                 365

Ala Ala Asp Pro His Glu Cys Tyr Ala Lys Val Phe Asp Glu Phe Lys
    370                 375                 380

Pro Leu Val Glu Glu Pro Gln Asn Leu Ile Lys Gln Asn Cys Glu Leu
385                 390                 395                 400

Phe Glu Gln Leu Gly Glu Tyr Lys Phe Gln Asn Ala Leu Leu Val Arg
                405                 410                 415

Tyr Thr Lys Lys Val Pro Gln Val Ser Thr Pro Thr Leu Val Glu Val
            420                 425                 430

Ser Arg Asn Leu Gly Lys Val Gly Ser Lys Cys Cys Lys His Pro Glu
        435                 440                 445

Ala Lys Arg Met Pro Cys Ala Glu Asp Tyr Leu Ser Val Val Leu Asn
    450                 455                 460

Gln Leu Cys Val Leu His Glu Lys Thr Pro Val Ser Asp Arg Val Thr
465                 470                 475                 480

Lys Cys Cys Thr Glu Ser Leu Val Asn Arg Arg Pro Cys Phe Ser Ala
                485                 490                 495

Leu Glu Val Asp Glu Thr Tyr Val Pro Lys Glu Phe Asn Ala Glu Thr
            500                 505                 510

Phe Thr Phe His Ala Asp Ile Cys Thr Leu Ser Glu Lys Glu Arg Gln
        515                 520                 525

Ile Lys Lys Gln Thr Ala Leu Val Glu Leu Val Lys His Lys Pro Lys
    530                 535                 540

Ala Thr Lys Glu Gln Leu Lys Ala Val Met Asp Asp Phe Ala Ala Phe
545                 550                 555                 560

Val Glu Lys Cys Cys Lys Ala Asp Asp Lys Glu Thr Cys Phe Ala Glu
                565                 570                 575

Glu Gly Lys Lys Leu Val Ala Ala Ser Gln Ala Ala Leu Gly Leu Gly
            580                 585                 590

Gly Gly Gly Ser Gly Gly Gly Gly Ser Gly Gly Gly Gly Ser Ala Asp
        595                 600                 605

Ser Ser Pro Leu Leu Gln Phe Gly Gly Gln Val Arg Gln Arg Tyr Leu
    610                 615                 620

Tyr Thr Asp Asp Ala Gln Arg Thr Glu Ala His Leu Glu Ile Arg Glu
625                 630                 635                 640

Asp Gly Thr Val Gly Gly Ala Ala Asp Gln Ser Pro Glu Ser Leu Leu
                645                 650                 655

Gln Leu Lys Ala Leu Lys Pro Gly Val Ile Gln Ile Leu Gly Val Arg
            660                 665                 670

Thr Pro Arg Phe Leu Cys Gln Arg Pro Asp Gly Ala Leu Tyr Gly Ser
        675                 680                 685

Leu His Phe Asp Pro Glu Ala Cys Ser Phe Arg Glu Leu Leu Leu Glu
    690                 695                 700

Asp Gly Tyr Asn Val Tyr Gln Ser Glu Ala His Gly Leu Pro Leu His
705                 710                 715                 720

Leu Pro Gly Asn Lys Ser Pro His Arg Asp Pro Ala Pro Arg Gly Pro
                725                 730                 735

Ala Arg Phe Leu Pro Leu Pro Phe Leu Pro Pro Ala Leu Pro Glu Pro
            740                 745                 750

Pro Gly Ile Leu Gly Pro Gln Pro Pro Asp Val Gly Ser Ser Asp Pro
        755                 760                 765

Leu Ser Met Val Gly Pro Ser Gln Gly Arg Ser Pro Ser Tyr Ala Ser
    770                 775                 780

<210>  4
<211>  398
<212>  PRT
<213>  Artificial Sequence

<220>
<223>  Protein translated from synthetic DNA

<400>  4

Ala Asp Ser Ser Pro Leu Leu Gln Phe Gly Gly Gln Val Arg Gln Arg
1               5                   10                  15

Tyr Leu Tyr Thr Asp Asp Ala Gln Arg Thr Glu Ala His Leu Glu Ile
            20                  25                  30

Arg Glu Asp Gly Thr Val Gly Gly Ala Ala Asp Gln Ser Pro Glu Ser
        35                  40                  45

Leu Leu Gln Leu Lys Ala Leu Lys Pro Gly Val Ile Gln Ile Leu Gly
    50                  55                  60

Val Arg Thr Pro Arg Phe Leu Cys Gln Arg Pro Asp Gly Ala Leu Tyr
65                  70                  75                  80

Gly Ser Leu His Phe Asp Pro Glu Ala Cys Ser Phe Arg Glu Leu Leu
                85                  90                  95

Leu Glu Asp Gly Tyr Asn Val Tyr Gln Ser Glu Ala His Gly Leu Pro
            100                 105                 110

Leu His Leu Pro Gly Asn Lys Ser Pro His Arg Asp Pro Ala Pro Arg
        115                 120                 125

Gly Pro Ala Arg Phe Leu Pro Leu Pro Phe Leu Pro Pro Ala Leu Pro
    130                 135                 140

Glu Pro Pro Gly Ile Leu Gly Pro Gln Pro Pro Asp Val Gly Ser Ser
145                 150                 155                 160

Asp Pro Leu Ser Met Val Gly Pro Ser Gln Gly Arg Ser Pro Ser Tyr
                165                 170                 175

Ala Ser Gly Gly Gly Gly Ser Gly Gly Gly Gly Ser Gly Gly Gly Gly
            180                 185                 190

Ser Val Glu Glu Pro Gln Asn Leu Ile Lys Gln Asn Cys Glu Leu Phe
        195                 200                 205

Glu Gln Leu Gly Glu Tyr Lys Phe Gln Asn Ala Leu Leu Val Arg Tyr
    210                 215                 220

Thr Lys Lys Val Pro Gln Val Ser Thr Pro Thr Leu Val Glu Val Ser
225                 230                 235                 240

Arg Asn Leu Gly Lys Val Gly Ser Lys Cys Cys Lys His Pro Glu Ala
                245                 250                 255

Lys Arg Met Pro Cys Ala Glu Asp Tyr Leu Ser Val Val Leu Asn Gln
            260                 265                 270

Leu Cys Val Leu His Glu Lys Thr Pro Val Ser Asp Arg Val Thr Lys
        275                 280                 285

Cys Cys Thr Glu Ser Leu Val Asn Arg Arg Pro Cys Phe Ser Ala Leu
    290                 295                 300

Glu Val Asp Glu Thr Tyr Val Pro Lys Glu Phe Asn Ala Glu Thr Phe
305                 310                 315                 320

Thr Phe His Ala Asp Ile Cys Thr Leu Ser Glu Lys Glu Arg Gln Ile
                325                 330                 335

Lys Lys Gln Thr Ala Leu Val Glu Leu Val Lys His Lys Pro Lys Ala
            340                 345                 350

Thr Lys Glu Gln Leu Lys Ala Val Met Asp Asp Phe Ala Ala Phe Val
        355                 360                 365

Glu Lys Cys Cys Lys Ala Asp Asp Lys Glu Thr Cys Phe Ala Glu Glu
    370                 375                 380

Gly Lys Lys Leu Val Ala Ala Ser Gln Ala Ala Leu Gly Leu
385                 390                 395

<210>  5
<211>  398
<212>  PRT
<213>  Artificial Sequence

<220>
<223>  Protein translated from synthetic DNA

<400>  5

Val Glu Glu Pro Gln Asn Leu Ile Lys Gln Asn Cys Glu Leu Phe Glu
1               5                   10                  15

Gln Leu Gly Glu Tyr Lys Phe Gln Asn Ala Leu Leu Val Arg Tyr Thr
            20                  25                  30

Lys Lys Val Pro Gln Val Ser Thr Pro Thr Leu Val Glu Val Ser Arg
        35                  40                  45

Asn Leu Gly Lys Val Gly Ser Lys Cys Cys Lys His Pro Glu Ala Lys
    50                  55                  60

Arg Met Pro Cys Ala Glu Asp Tyr Leu Ser Val Val Leu Asn Gln Leu
65                  70                  75                  80

Cys Val Leu His Glu Lys Thr Pro Val Ser Asp Arg Val Thr Lys Cys
                85                  90                  95

Cys Thr Glu Ser Leu Val Asn Arg Arg Pro Cys Phe Ser Ala Leu Glu
        100                 105                 110

Val Asp Glu Thr Tyr Val Pro Lys Glu Phe Asn Ala Glu Thr Phe Thr
    115                 120                 125

Phe His Ala Asp Ile Cys Thr Leu Ser Glu Lys Glu Arg Gln Ile Lys
    130                 135                 140

Lys Gln Thr Ala Leu Val Glu Leu Val Lys His Lys Pro Lys Ala Thr
145                 150                 155                 160

Lys Glu Gln Leu Lys Ala Val Met Asp Asp Phe Ala Ala Phe Val Glu
                165                 170                 175

Lys Cys Cys Lys Ala Asp Asp Lys Glu Thr Cys Phe Ala Glu Glu Gly
        180                 185                 190

Lys Lys Leu Val Ala Ala Ser Gln Ala Ala Leu Gly Leu Gly Gly Gly
    195                 200                 205

Gly Ser Gly Gly Gly Gly Ser Gly Gly Gly Gly Ser Ala Asp Ser Ser
    210                 215                 220

Pro Leu Leu Gln Phe Gly Gly Gln Val Arg Gln Arg Tyr Leu Tyr Thr
225                 230                 235                 240

Asp Asp Ala Gln Arg Thr Glu Ala His Leu Glu Ile Arg Glu Asp Gly
                245                 250                 255

Thr Val Gly Gly Ala Ala Asp Gln Ser Pro Glu Ser Leu Leu Gln Leu
        260                 265                 270

Lys Ala Leu Lys Pro Gly Val Ile Gln Ile Leu Gly Val Arg Thr Pro
    275                 280                 285

Arg Phe Leu Cys Gln Arg Pro Asp Gly Ala Leu Tyr Gly Ser Leu His
    290                 295                 300

Phe Asp Pro Glu Ala Cys Ser Phe Arg Glu Leu Leu Leu Glu Asp Gly
305                 310                 315                 320

Tyr Asn Val Tyr Gln Ser Glu Ala His Gly Leu Pro Leu His Leu Pro
                325                 330                 335

Gly Asn Lys Ser Pro His Arg Asp Pro Ala Pro Arg Gly Pro Ala Arg
        340                 345                 350

Phe Leu Pro Leu Pro Phe Leu Pro Pro Ala Leu Pro Glu Pro Pro Gly
        355                 360                 365

Ile Leu Gly Pro Gln Pro Pro Asp Val Gly Ser Ser Asp Pro Leu Ser
    370                 375                 380

Met Val Gly Pro Ser Gln Gly Arg Ser Pro Ser Tyr Ala Ser
385                 390                 395

<210>  6
<211>  537
<212>  DNA
<213>  Artificial Sequence

<220>
<223>  Synthetic DNA

<400>  6
gcagactcca gtcctctcct gcaattcggg ggccaagtcc ggcagcggta cctctacaca      60

gatgatgccc agcgtacaga agcccacctg gagatcaggg aggatgggac ggtggggggc     120

gctgctgacc agagccccga aagtctcctg cagctgaaag ccttgaagcc gggagttatt     180

caaatcttgg gagtccgtac accgaggttc ctgtgccagc ggccagatgg ggccctgtat     240

ggatcgctcc actttgaccc tgaggcctgc agcttccggg agctgcttct tgaggacgga     300

tacaatgttt accagtccga agcccacggc ctcccgctgc acctgccagg gaacaagtcc     360

ccacaccggg accctgcacc ccgaggacca gctcgcttcc tgccactacc attcctgccc     420

cccgcactcc cggagccacc cggaatcctg ggtccccagc cccccgatgt gggctcctcg     480

gaccctctga gcatggtggg accttcccag ggccgaagcc ccagctacgc ttcctga       537

<210>  7
<211>  2355
<212>  DNA
<213>  Artificial Sequence

<220>
<223>  Synthetic DNA

<400>  7
gcagactcca gtcctctcct gcaattcggg ggccaagtcc ggcagcggta cctctacaca      60

gatgatgccc agcgtacaga agcccacctg gagatcaggg aggatgggac ggtggggggc     120

gctgctgacc agagccccga aagtctcctg cagctgaaag ccttgaagcc gggagttatt     180

caaatcttgg gagtccgtac accgaggttc ctgtgccagc ggccagatgg ggccctgtat     240

ggatcgctcc actttgaccc tgaggcctgc agcttccggg agctgcttct tgaggacgga     300

tacaatgttt accagtccga agcccacggc ctcccgctgc acctgccagg gaacaagtcc     360

ccacaccggg accctgcacc ccgaggacca gctcgcttcc tgccactacc attcctgccc     420

cccgcactcc cggagccacc cggaatcctg ggtccccagc cccccgatgt gggctcctcg 480

gaccctctga gcatggtggg accttcccag ggccgaagcc ccagctacgc ttccggtggt 540

ggtggttctg gtggtggtgg ttctggtggt ggtggttcta gaggtgtttt cagaagagac 600

gctcacaagt ctgaagttgc tcacagattc aaggacttgg gtgaagaaaa cttcaaggct 660

ttggttttga tcgctttcgc tcaatacttg caacaatgtc cattcgaaga ccacgttaag 720

ttggttaacg aagttactga atttgctaag acttgtgttg ctgacgaatc tgctgaaaac 780

tgtgacaagt ctttgcacac tttgttcggt gacaagttgt gtactgttgc tactttgaga 840

gaaacttacg gtgaaatggc tgactgttgt gctaagcaag aaccagaaag aaacgaatgt 900

ttcttgcaac acaaggacga caacccaaac ttgccaagat tggttagacc agaagttgac 960

gttatgtgta ctgctttcca cgacaacgaa gaaactttct tgaagaagta cttgtacgaa 1020

atcgctagaa gacacccata cttctacgct ccagaattgt tgttcttcgc taagagatac 1080

aaggctgctt tcactgaatg ttgtcaagct gctgacaagg ctgcttgttt gttgccaaag 1140

ttggacgaat tgagagacga aggtaaggct tcttctgcta agcaaagatt gaagtgtgct 1200

tctttgcaaa agttcggtga aagagctttc aaggcttggg ctgttgctag attgtctcaa 1260

agattcccaa aggctgaatt tgctgaagtt tctaagttgg ttactgactt gactaaggtt 1320

cacactgaat gttgtcacgg tgacttgttg gaatgtgctg acgacagagc tgacttggct 1380

aagtacatct gtgaaaacca agactctatc tcttctaagt tgaaggaatg ttgtgaaaag 1440

ccattgttgg aaaagtctca ctgtatcgct gaagttgaaa acgacgaaat gccagctgac 1500

ttgccatctt tggctgctga cttcgttgaa tctaaggacg tttgtaagaa ctacgctgaa 1560

gctaaggacg ttttcttggg tatgttcttg tacgaatacg ctagaagaca cccagactac 1620

tctgttgttt tgttgttgag attggctaag acttacgaaa ctactttgga aaagtgttgt 1680

gctgctgctg acccacacga atgttacgct aaggttttcg acgaatttaa gccattggtt 1740

gaagaaccac aaaacttgat caagcaaaac tgtgaattgt tcgaacaatt gggtgaatac 1800

aagttccaaa acgctttgtt ggttagatac actaagaagg ttccacaagt ttctactcca 1860

actttggttg aagtttctag aaacttgggt aaggttggtt ctaagtgttg taagcaccca 1920

gaagctaaga gaatgccatg tgctgaagac tacttgtctg ttgttttgaa ccaattgtgt 1980

gttttgcacg aaaagactcc agtttctgac agagttacta agtgttgtac tgaatctttg 2040

gttaacagaa gaccatgttt ctctgctttg gaagttgacg aaacttacgt tccaaaggaa 2100

tttaacgctg aaactttcac tttccacgct gacatctgta ctttgtctga aaaggaaaga 2160

caaatcaaga agcaaactgc tttggttgaa ttggttaagc acaagccaaa ggctactaag 2220

gaacaattga aggctgttat ggacgacttc gctgctttcg ttgaaaagtg ttgtaaggct 2280

gacgacaagg aaacttgttt cgctgaagaa ggtaagaagt tggttgctgc ttctcaagct 2340

gctttgggtt tgtga 2355

<210> 8
<211> 2355
<212> DNA
<213> Artificial Sequence

<220>
<223> Synthetic DNA

<400> 8
agaggtgttt tcagaagaga cgctcacaag tctgaagttg ctcacagatt caaggacttg 60

ggtgaagaaa acttcaaggc tttggttttg atcgctttcg ctcaatactt gcaacaatgt 120

ccattcgaag accacgttaa gttggttaac gaagttactg aatttgctaa gacttgtgtt 180

gctgacgaat ctgctgaaaa ctgtgacaag tctttgcaca ctttgttcgg tgacaagttg 240

tgtactgttg ctactttgag agaaacttac ggtgaaatgg ctgactgttg tgctaagcaa 300

gaaccagaaa gaaacgaatg tttcttgcaa cacaaggacg acaacccaaa cttgccaaga 360

ttggttagac cagaagttga cgttatgtgt actgctttcc acgacaacga agaaactttc 420

ttgaagaagt acttgtacga aatcgctaga agacacccat acttctacgc tccagaattg 480

ttgttcttcg ctaagagata caaggctgct ttcactgaat gttgtcaagc tgctgacaag 540

gctgcttgtt tgttgccaaa gttggacgaa ttgagagacg aaggtaaggc ttcttctgct 600

aagcaaagat tgaagtgtgc ttctttgcaa aagttcggtg aaagagcttt caaggcttgg 660

gctgttgcta gattgtctca aagattccca aaggctgaat ttgctgaagt ttctaagttg 720

gttactgact tgactaaggt tcacactgaa tgttgtcacg gtgacttgtt ggaatgtgct 780

gacgacagag ctgacttggc taagtacatc tgtgaaaacc aagactctat ctcttctaag 840

ttgaaggaat gttgtgaaaa gccattgttg gaaaagtctc actgtatcgc tgaagttgaa 900

aacgacgaaa tgccagctga cttgccatct ttggctgctg acttcgttga atctaaggac 960

gtttgtaaga actacgctga agctaaggac gttttcttgg gtatgttctt gtacgaatac 1020

gctagaagac acccagacta ctctgttgtt ttgttgttga gattggctaa gacttacgaa 1080

actactttgg aaaagtgttg tgctgctgct gacccacacg aatgttacgc taaggttttc 1140

gacgaattta agccattggt tgaagaacca caaaacttga tcaagcaaaa ctgtgaattg 1200

ttcgaacaat tgggtgaata caagttccaa aacgctttgt tggttagata cactaagaag 1260

gttccacaag tttctactcc aactttggtt gaagtttcta gaaacttggg taaggttggt 1320

tctaagtgtt gtaagcaccc agaagctaag agaatgccat gtgctgaaga ctacttgtct 1380

gttgttttga accaattgtg tgttttgcac gaaaagactc cagtttctga cagagttact 1440

aagtgttgta ctgaatcttt ggttaacaga agaccatgtt tctctgcttt ggaagttgac 1500

gaaacttacg ttccaaagga atttaacgct gaaactttca ctttccacgc tgacatctgt 1560

actttgtctg aaaaggaaag acaaatcaag aagcaaactg ctttggttga attggttaag 1620

cacaagccaa aggctactaa ggaacaattg aaggctgtta tggacgactt cgctgctttc 1680

gttgaaaagt gttgtaaggc tgacgacaag gaaacttgtt tcgctgaaga aggtaagaag 1740

ttggttgctg cttctcaagc tgctttgggt ttgggtggtg gtggttctgg tggtggtggt 1800

tctggtggtg gtggttctgc agactccagt cctctcctgc aattcggggg ccaagtccgg 1860

cagcggtacc tctacacaga tgatgcccag cgtacagaag cccacctgga gatcagggag 1920

gatgggacgg tggggggcgc tgctgaccag agccccgaaa gtctcctgca gctgaaagcc 1980

ttgaagccgg gagttattca aatcttggga gtccgtacac cgaggttcct gtgccagcgg 2040

ccagatgggg ccctgtatgg atcgctccac tttgaccctg aggcctgcag cttccgggag 2100

ctgcttcttg aggacggata caatgtttac cagtccgaag cccacggcct cccgctgcac 2160

ctgccaggga acaagtcccc acaccgggac cctgcacccc gaggaccagc tcgcttcctg 2220

ccactaccat tcctgccccc cgcactcccg gagccacccg gaatcctggg tccccagccc 2280

cccgatgtgg gctcctcgga ccctctgagc atggtgggac cttcccaggg ccgaagcccc 2340

agctacgctt cctga 2355

<210> 9
<211> 1197
<212> DNA
<213> Artificial Sequence

<220>
<223> Synthetic DNA

<400> 9
gcagactcca gtcctctcct gcaattcggg ggccaagtcc ggcagcggta cctctacaca 60

gatgatgccc agcgtacaga agcccacctg gagatcaggg aggatgggac ggtggggggc 120

gctgctgacc agagccccga aagtctcctg cagctgaaag ccttgaagcc gggagttatt 180

caaatcttgg gagtccgtac accgaggttc ctgtgccagc ggccagatgg ggccctgtat 240

ggatcgctcc actttgaccc tgaggcctgc agcttccggg agctgcttct tgaggacgga 300

tacaatgttt accagtccga agcccacggc ctcccgctgc acctgccagg gaacaagtcc 360

ccacaccggg accctgcacc ccgaggacca gctcgcttcc tgccactacc attcctgccc 420

cccgcactcc cggagccacc cggaatcctg ggtccccagc cccccgatgt gggctcctcg 480

gaccctctga gcatggtggg accttcccag ggccgaagcc ccagctacgc ttccggtggt 540

ggtggttctg gtggtggtgg ttctggtggt ggtggttctg ttgaagaacc acaaaacttg 600

atcaagcaaa actgtgaatt gttcgaacaa ttgggtgaat acaagttcca aaacgctttg 660

ttggttagat acactaagaa ggttccacaa gtttctactc caactttggt tgaagtttct 720

agaaacttgg gtaaggttgg ttctaagtgt tgtaagcacc cagaagctaa gagaatgcca 780

tgtgctgaag actacttgtc tgttgttttg aaccaattgt gtgttttgca cgaaaagact 840

ccagtttctg acagagttac taagtgttgt actgaatctt tggttaacag aagaccatgt 900

ttctctgctt tggaagttga cgaaacttac gttccaaagg aattcaacgc tgaaactttc 960

actttccacg ctgacatctg tactttgtct gaaaaggaaa gacaaatcaa gaagcaaact 1020

gctttggttg aattggttaa gcacaagcca aaggctacta aggaacaatt gaaggctgtt 1080

atggacgact tcgctgcttt cgttgaaaag tgttgtaagg ctgacgacaa ggaaacttgt 1140

ttcgctgaag aaggtaagaa gttggttgct gcttctcaag ctgctttggg tttgtaa 1197

<210> 10
<211> 1197
<212> DNA
<213> Artificial Sequence

<220>
<223> Synthetic DNA

<400> 10
gttgaagaac cacaaaactt gatcaagcaa aactgtgaat tgttcgaaca attgggtgaa 60

tacaagttcc aaaacgcttt gttggttaga tacactaaga aggttccaca agtttctact 120

ccaactttgg ttgaagtttc tagaaacttg ggtaaggttg gttctaagtg ttgtaagcac 180

ccagaagcta agagaatgcc atgtgctgaa gactacttgt ctgttgtttt gaaccaattg 240

tgtgttttgc acgaaaagac tccagtttct gacagagtta ctaagtgttg tactgaatct 300

ttggttaaca gaagaccatg tttctctgct ttggaagttg acgaaactta cgttccaaag 360

gaattcaacg ctgaaacttt cactttccac gctgacatct gtactttgtc tgaaaaggaa 420

agacaaatca agaagcaaac tgctttggtt gaattggtta agcacaagcc aaaggctact 480

aaggaacaat tgaaggctgt tatggacgac ttcgctgctt tcgttgaaaa gtgttgtaag 540

gctgacgaca aggaaacttg tttcgctgaa gaaggtaaga agttggttgc tgcttctcaa 600

gctgctttgg gtttgggtgg tggtggttct ggtggtggtg gttctggtgg tggtggttct 660

gcagactcca gtcctctcct gcaattcggg ggccaagtcc ggcagcggta cctctacaca 720

gatgatgccc agcgtacaga agcccacctg gagatcaggg aggatgggac ggtggggggc 780

gctgctgacc agagccccga aagtctcctg cagctgaaag ccttgaagcc gggagttatt 840

caaatcttgg gagtccgtac accgaggttc ctgtgccagc ggccagatgg ggccctgtat 900

ggatcgctcc actttgaccc tgaggcctgc agcttccggg agctgcttct tgaggacgga 960

tacaatgttt accagtccga agcccacggc ctcccgctgc acctgccagg gaacaagtcc 1020

ccacaccggg accctgcacc ccgaggacca gctcgcttcc tgccactacc attcctgccc 1080

cccgcactcc cggagccacc cggaatcctg ggtccccagc cccccgatgt gggctcctcg 1140

gaccctctga gcatggtggg accttcccag ggccgaagcc ccagctacgc ttcctaa 1197

<210> 11
<211> 178
<212> PRT
<213> Artificial Sequence

<220>
<223> Protein translated from synthetic DNA

<400> 11

Met Arg Asp Ser Ser Pro Leu Val His Tyr Gly Trp Gly Asp Pro Ile
1               5                   10                  15

Arg Leu Arg His Leu Tyr Thr Ser Gly Pro His Gly Leu Ser Ser Cys
            20                  25                  30

Phe Leu Arg Ile Arg Ala Asp Gly Val Val Asp Cys Ala Arg Gly Gln
        35                  40                  45

Ser Ala His Ser Leu Leu Glu Ile Lys Ala Val Ala Leu Arg Thr Val
    50                  55                  60

Ala Ile Lys Gly Val His Ser Val Arg Tyr Leu Cys Met Gly Ala Asp
65                  70                  75                  80

Gly Lys Met Gln Gly Leu Leu Gln Tyr Ser Glu Glu Asp Cys Ala Phe
                85                  90                  95

Glu Glu Glu Ile Arg Pro Asp Gly Tyr Asn Val Tyr Arg Ser Glu Lys
            100                 105                 110

His Arg Leu Pro Val Cys Leu Ser Ser Ala Lys Gln Arg Gln Leu Tyr
        115                 120                 125

Lys Asn Arg Gly Phe Leu Pro Leu Pro Gly Leu Pro Pro Ala Leu Pro
    130                 135                 140

Glu Pro Pro Gly Ile Leu Ala Pro Gln Pro Pro Asp Val Gly Ser Ser
145                 150                 155                 160

Asp Pro Leu Ser Met Val Gly Pro Ser Gln Gly Arg Ser Pro Ser Tyr
                165                 170                 175

Ala Ser

<210>  12
<211>  189
<212>  PRT
<213>  Artificial Sequence

<220>
<223>  Protein translated from synthetic DNA

<400>  12

Met Arg Asp Ser Ser Pro Leu Val His Tyr Gly Trp Gly Asp Pro Ile
1               5                   10                  15

Arg Leu Arg His Leu Tyr Thr Ser Gly Pro His Gly Leu Ser Ser Cys
            20                  25                  30

Phe Leu Arg Ile Arg Ala Asp Gly Val Val Asp Cys Ala Arg Gly Gln
        35                  40                  45

Ser Ala His Ser Leu Leu Glu Ile Lys Ala Val Ala Leu Arg Thr Val
    50                  55                  60

Ala Ile Lys Gly Val His Ser Val Arg Tyr Leu Cys Met Gly Ala Asp
65                  70                  75                  80

Gly Lys Met Gln Gly Leu Leu Gln Tyr Ser Glu Glu Asp Cys Ala Phe
                85                  90                  95

Glu Glu Glu Ile Arg Pro Asp Gly Tyr Asn Val Tyr Arg Ser Glu Lys
            100                 105                 110

His Arg Leu Pro Val Cys Leu Ser Ser Ala Lys Gln Arg Gln Leu Tyr
        115                 120                 125

Lys Asn Arg Gly Phe Leu Pro Leu Cys His Phe Leu Pro Met Leu Pro
    130                 135                 140

Met Val Pro Glu Glu Pro Glu Asp Leu Arg Gly His Leu Glu Ser Asp
145                 150                 155                 160

Met Phe Ser Ser Pro Pro Asp Val Gly Ser Ser Asp Pro Leu Ser Met
                165                 170                 175

Val Gly Pro Ser Gln Gly Arg Ser Pro Ser Tyr Ala Ser
            180                 185


<210>  13
<211>  221
<212>  PRT
<213>  Artificial Sequence

<220>
<223>  Protein translated from synthetic DNA

<400>  13

Met Asp Ser Asp Glu Thr Gly Phe Glu His Ser Gly Leu Trp Val Ser
1               5                   10                  15

Val Leu Ala Gly Leu Leu Leu Gly Ala Cys Gln Ala His Pro Ile Pro
            20                  25                  30

Asp Ser Ser Pro Leu Leu Gln Phe Gly Gly Gln Val Arg Gln Arg Tyr
        35                  40                  45

Leu Tyr Thr Asp Asp Ala Gln Gln Thr Glu Ala His Leu Glu Ile Arg
    50                  55                  60

Glu Asp Gly Thr Val Gly Gly Ala Ala Asp Gln Ser Pro Glu Ser Leu
65                  70                  75                  80

Leu Gln Leu Lys Ala Leu Lys Pro Gly Val Ile Gln Ile Leu Gly Val
                85                  90                  95

Lys Thr Ser Arg Phe Leu Cys Gln Arg Pro Asp Gly Ala Leu Tyr Gly
            100                 105                 110

Ser Leu His Phe Asp Pro Glu Ala Cys Ser Phe Arg Glu Leu Leu Leu
        115                 120                 125

Glu Asp Gly Tyr Asn Val Tyr Gln Ser Glu Ala His Gly Leu Pro Leu
    130                 135                 140

His Leu Pro Gly Asn Lys Ser Pro His Arg Asp Pro Ala Pro Arg Gly
145                 150                 155                 160

Pro Ala Arg Phe Leu Pro Leu Cys His Phe Leu Pro Met Leu Pro Met
                165                 170                 175

Val Pro Glu Glu Pro Glu Asp Leu Arg Gly His Leu Glu Ser Asp Met
            180                 185                 190

Phe Ser Ser Pro Leu Glu Thr Asp Ser Met Asp Pro Phe Gly Leu Val
        195                 200                 205

Thr Gly Leu Glu Ala Val Arg Ser Pro Ser Phe Glu Lys
    210                 215                 220

<210>  14

<211>  210
<212>  PRT
<213>  Artificial Sequence

<220>
<223>  Protein translated from synthetic DNA

<400>  14

Met Asp Ser Asp Glu Thr Gly Phe Glu His Ser Gly Leu Trp Val Ser
1               5                   10                  15

Val Leu Ala Gly Leu Leu Leu Gly Ala Cys Gln Ala His Pro Ile Pro
            20                  25                  30

Asp Ser Ser Pro Leu Leu Gln Phe Gly Gly Gln Val Arg Gln Arg Tyr
        35                  40                  45

Leu Tyr Thr Asp Asp Ala Gln Gln Thr Glu Ala His Leu Glu Ile Arg
    50                  55                  60

Glu Asp Gly Thr Val Gly Gly Ala Ala Asp Gln Ser Pro Glu Ser Leu
65                  70                  75                  80

Leu Gln Leu Lys Ala Leu Lys Pro Gly Val Ile Gln Ile Leu Gly Val
                85                  90                  95

Lys Thr Ser Arg Phe Leu Cys Gln Arg Pro Asp Gly Ala Leu Tyr Gly
            100                 105                 110

Ser Leu His Phe Asp Pro Glu Ala Cys Ser Phe Arg Glu Leu Leu Leu
        115                 120                 125

Glu Asp Gly Tyr Asn Val Tyr Gln Ser Glu Ala His Gly Leu Pro Leu
    130                 135                 140

His Leu Pro Gly Asn Lys Ser Pro His Arg Asp Pro Ala Pro Arg Gly
145                 150                 155                 160

Pro Ala Arg Phe Leu Pro Leu Pro Gly Leu Pro Pro Ala Leu Pro Glu
                165                 170                 175

Pro Pro Gly Ile Leu Ala Pro Gln Pro Leu Glu Thr Asp Ser Met Asp
            180                 185                 190

Pro Phe Gly Leu Val Thr Gly Leu Glu Ala Val Arg Ser Pro Ser Phe
        195                 200                 205

Glu Lys
    210

## Description

### Technical Field

The present invention relates to the application of FGF21 in the preparation of medicines for treating colorectal cancer, in particular to the application of human FGF21 in the preparation of medicines for treating colorectal cancer, and belongs to the technical field of medicine.

### Background of the Invention

Colorectal cancer is one of the common malignant tumors in clinical practice. Colorectal cancer includes colon cancer and rectal cancer and is the third most common malignant tumor in China. The incidence rate is increasing with the improvement of living standards. With 1023152 new cases each year around the world, colorectal cancer ranks third in the incidence of common malignant tumors, accounting for 10.38% of the total incidence of malignant tumors. In my country, with the changes in residents' lifestyles and diet, the incidence of colorectal cancer is increasing at an annual rate of 4%, and the average annual growth rate is 2% higher than that of the world.

Currently, the treatment of colorectal cancer is mainly based on radical surgery, with chemotherapy, radiotherapy, traditional Chinese medicine treatment and biological treatment as its auxiliary treatment means. Approximately two-thirds of patients diagnosed for the first time underwent radical surgery, and more than half of the patients experienced recurrence or distant metastasis. For advanced cancer or cancer with extensive metastasis, radical surgery cannot be performed, and comprehensive medical treatment often becomes the main treatment means. Among operable patients, the recurrence rate is still higher after colorectal cancer surgery. The commonly used therapeutic drugs are usually broad-spectrum chemical drugs with many side effects. Therefore, the development of a safe drug for treating colorectal cancer that has the effect of inhibiting tumor growth and recurrence as well as the organ protection effect without related obvious side effects is the ideal goal of current research and development.

In recent years, a large number of clinical investigations have shown that the occurrence and development of colorectal cancer are closely related to metabolic abnormalities. A large number of studies have found that metabolic pathways including glycolysis, tricarboxylic acid cycle, fatty acid metabolism, glutamine metabolism and the like have undergone reprogramming changes in tumor cells, and tumor cells can provide fatty acids, nucleotides and the like required for proliferation by coordinating various metabolic pathways, such that the synthesis process of biological macromolecules is more suitable for the rapid proliferation of tumor cells. In particular, it should be pointed out that metabolic abnormalities are not only the result of tumor occurrence and development, but may also be directly involved in the initiation process of tumors. Currently, the development of anti-tumor drugs from the perspective of metabolism has been successfully applied in many tumors. For example, recent studies have found that some genes encoding metabolic enzymes are oncogenes themselves, for example, the mutation of fumarate carboxylase is related to the occurrence and development of renal cancer; the mutation of succinate carboxylase is related to the occurrence and development of pheochromocytoma; the mutation of isocitrate dehydrogenase produces metabolites of small molecules 2HG, which inhibits the expression of HNF4a and thus induces cholangiocarcinoma and the like. Based on the above analysis, it is indicated that targeting tumor cell metabolism may be one of the effective ways to kill tumor cells. However, the drugs currently developed are all small molecule inhibitors with poor targeting and more side effects, while bioprotein drugs are absorbed quickly and have strong targeting. Therefore, the development of new targeted bioprotein preparations for colorectal cancer is currently a hot spot in tumor research.

### Summary of the Invention

The present invention has discovered through a large number of studies that human fibroblast growth factor 21, which is an important member involved in multiple links of the glycolipid metabolism network, can significantly inhibit the occurrence, development and metastasis of colorectal cancer. Based on this, the present invention discloses a new application of FGF21 protein in the preparation of medicines for preventing, relieving and/or treating colorectal cancer.

The present invention discloses the application of FGF21 protein in the preparation of medicines for preventing, relieving and/or treating colorectal cancer.

In one implementation, the amino acid sequence of the FGF21 protein is as set forth in any one of SEQ ID NO. 1-5 or SEQ ID NO. 11-14.

In one implementation, the effective dose of the FGF21 protein is 0.1-10 mg/kg.

The present invention discloses a medicine for treating colorectal cancer, and the active ingredient of the medicine includes FGF21 protein.

In one implementation, the amino acid sequence of the FGF21 protein is as set forth in any one of SEQ ID NO. 1-5 or SEQ ID NO. 11-14.

In one implementation, the medicine further includes a pharmaceutically acceptable carrier or accessory.

In one implementation, the administration route of the medicine includes oral, intraperitoneal injection, subcutaneous injection, intravenous injection or intramuscular injection.

The present invention discloses the application of FGF21 gene as a medicine target in the screen of medicines for treating colorectal cancer.

In one implementation, the nucleotide sequence of the FGF21 gene is as set forth in any one of SEQ ID NO. 6-10.

In one implementation, the application is for screening small molecule activators of FGF21 protein.

The present invention discloses the application of FGF21 protein in the preparation of inhibitors of sugar metabolism and/or glutamine metabolism in tumor.

In one implementation, the application is for preparing the inhibitors of one or more of HK2, PKM2, PFKP, PD, and GPD that are key proteins for inhibiting the sugar metabolism in tumor.

In one implementation, the application is for preparing an inhibitor of GLS, a protein related to glutamine metabolism.

The present invention discloses that the application of FGF21 protein in the preparation of medicines for preventing, relieving and/or treating one or more diseases of prostate cancer, esophageal cancer, colorectal adenocarcinoma, cervical cancer, endometrial cancer, ovarian cancer, pancreatic cancer, gallbladder cancer, liver cancer, clear cell kidney cancer, melanoma, and multiple myeloma.

**The present invention has the following beneficial effects compared with the prior art:**
(1) the present invention has discovered new functions of FGF21 gene, including the ability to significantly change tumor metabolism, especially significantly inhibit the key proteins HK2, PKM2 and GLS in the process of sugar metabolism and glutamine metabolism in tumor, and inhibit the proliferation, invasion and migration of colorectal tumor cell lines; and the ability to significantly reduce the size of tumor lesions in a mouse model, so that it can be used as an active ingredient in various dosage forms of drugs, and is of great significance for the treatment of colorectal cancer.
(2) the present invention can relieve the occurrence and development of colorectal cancer by injecting FGF21 protein, and inhibit the generation and metastasis of colon cancer.

### Brief Description of Figures

Figure 1 shows the effect of FGF21 on the growth rate of human colorectal cancer xenografts; where A is the change in tumor volume; and B is the change in tumor quality.
Figure 2 shows the effect of FGF21 AOM/DSS-induced colorectal cancer in mice; where A is the number of large intestine tumors; B is the morphology of the large intestine.
Figure 3 shows the effect of FGF21 on the key protein of tumor metabolism; where A is the change in the key protein of sugar metabolism pathway in tumor; B is the change in the key protein of glutamine metabolism in tumor.

### Detailed Description of Embodiments

Experimental animals and feeding: Nude mice and C57BU6 mice were purchased from Shanghai SLAC. They were raised in the Animal Center in Wuxi School of Medicine, Jiangnan University under a 12-hour light/dark cycle at a temperature of 20±2°C.

Cell culture: Colorectal cancer cell line SW620 was provided by the Institute of Biochemistry and Cell Biology, Chinese Academy of Sciences;

RPM11640, DMEM, and 0.05% Trypsin were purchased from Boster Company; fetal bovine serum was purchased from Sijiqing Comany. Azoxymethane was purchased from Sigma Company (batch number 098k1488), Dextran Sulfate Sodium MP Biomedicals (batch number 0216011050); other drugs were domestically produced analytically pure. Colorectal cancer cell lines were grown adherently in RPMI 1640 culture medium containing 10% fetal bovine serum, cultured in a humidified incubator at 37°C and 5% CO₂, and passaged every other day.

### Embodiment 1: Application of FGF21 in the preparation of medicines

FGF21 protein was prepared by culturing microbial cells, The preparation method has been disclosed in the patent application with the authorized announcement number CN 106432509 B.

### Embodiment 2: Effect of FGF21 on the growth rate of human colorectal cancer xenografts

FGF21 protein with the amino acid sequence as set forth in SEQ ID NO. 1 was prepared according to the method of Embodiment 1.

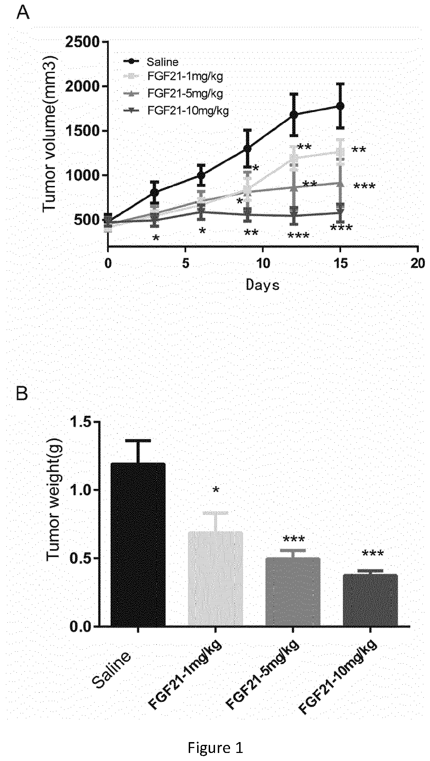

SW620 cells, human colon cancer cells, were subcutaneously inoculated into the 6-week-old male nude mice at 1×10⁶ cells/mouse. When the tumor grew to 200 mm³, they were randomly divided into four groups: (1) normal saline group: injection with the same volume of normal saline as the experimental group; (2) low-dose group: injection with 1 mg/kg FGF21; (3) medium-dose group: injection with 5 mg/kg FGF21; and (4) high-dose group: injection with 10 mg/kg FGF21. The mice were injected once a day for 15 consecutive days. The tumor volume was monitored every day, the mice were sacrificed three weeks later, and the tumor weight was weighed. The results showed that the three different doses of FGF21 could inhibit the xenograft volume and the final tumor weight in a dose-dependent manner. After injecting with high-dose of FGF21 (10 mg/kg), it can completely inhibit tumor growth and maintain the tumor volume at 500 mm³, the final volume was only 30% of the control group, Similarly, the tumor weight had also reduced by about 70%. Injection with 1 mg/kg and 5 mg/kg can reduce tumor weight by 30% and 50%, respectively (as shown in Figure 1).

### Embodiment 3: Effect of FGF21 on AOM/DSS-induced colorectal cancer in mice

FGF21 protein with the amino acid sequence as set forth in SEQ ID NO. 1 was prepared according to the method of Embodiment 1.

C57 mice were injected with 10 mg/kg of azoxymethane in the first week and had a free access cycle to 2.5% dextran sulfate sodium at the same time. Each cycle consisted of three weeks (21 days). The mice drank water containing dextran sulfate sodium in the first week and ordinary water in the next two weeks for a total of three cycles with 3 weeks per cycle. At the sixth week, they were divided into normal saline group and FGF21 injection group. The FGF21 injection group was injected at a dose of 10 mg/kg. The normal saline group was injected with the same amount of normal saline once a day for 4 consecutive weeks.

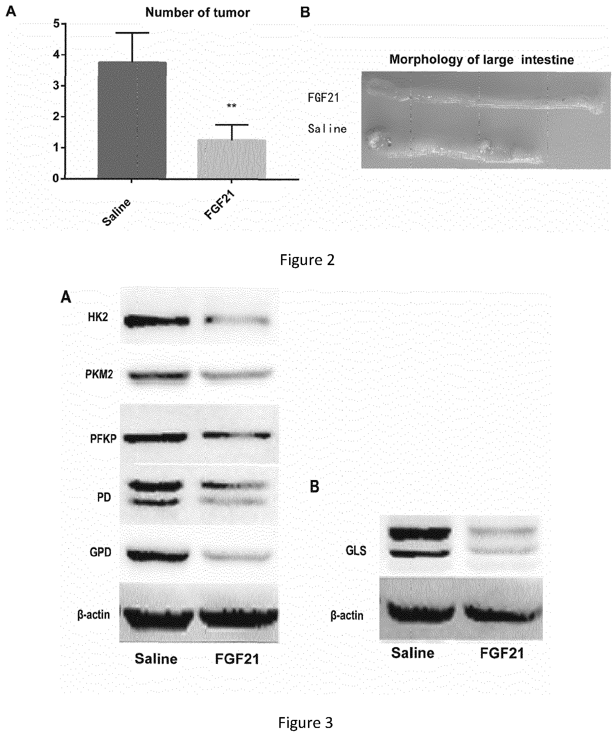

The results were shown in Figure 2A. After injection of FGF21, the average number of tumor lesions can be reduced from 4 to 1. Figure 2B showed typical pictures of the large intestine for the FGF21 treatment group and the control group, which can intuitively show the therapeutic effect of FGF21 including reducing the number of tumors, improving the problem of shortened colon length, and increasing the length of the colon by 10-20 mm compared with the control group with an average increase of 18 mm.

### Embodiment 4: Effect of FGF21 on key proteins in tumor metabolism

FGF21 protein with the amino acid sequence as set forth in SEQ ID NO. 1 was prepared according to the method of Embodiment 1.

The level of tumor metabolism-related proteins in the tumor tissues of Embodiment 3 was detected by Western Blot (protein immunoblotting):
a) Formulation of solution:
   (1) 10% (w/v) sodium dodecyl sulfate (SDS) solution: 0.1 g of SDS and 1 ml H₂O deionized water was used for preparation, and the solution was stored at room temperature.
   (2) separation gel buffer: 1.5 mmol/L Tris-HCL (pH 8.8): 18.15 g Tris was dissolved in 80 ml of water, adjusted to pH 8.8 with HCI, and diluted with water to a final volume of 100 ml.
   (3) concentrated gel buffer: 0.5 mmol/L Tris-HCL (pH 6.8): 6.05 g Tris was dissolved in 80 ml of water, adjusted to pH 6.8 with HCI, and diluted with water to a final volume of 100 ml.
   (4) SDS-PAGE loading buffer: 8 ml of 0.5 mol/L Tris buffer at pH 6.8, 6.4 ml glycerol, 12.8 ml of 10% SDS, 3.2 ml of mercaptoethanol, 1.6 ml of 0.05% bromophenol blue, and 32 ml of H₂O were mixed well for later use.
   (5) Tris-glycine electrophoresis buffer: 303 g Tris, 188 g glycine and 10 g SDS were dissolved in distilled water to 1000 ml, and diluted 10 times before use.
   (6) membrane transfer buffer: 14.4 g glycine and 6.04 g Tris were weighed, added with 200 ml methanol, and added with water to a total volume of 1 L.
   (7) Tris buffered salt solution (TBS): 20 mmol/L Tris/HCI (pH 7.5) and 500 mmol/L NaCl.
b) The specific steps were as follows: part of the transplanted tumor cells in Embodiment 2 was taken and washed 3 times with PBS, the lysate was added, the mixture was boiled directly for 5 min, cooled on ice, and centrifuged at 12000 rpm for 2 min, and the supernatant taken and stored at -20°C for later use.

(A) Determination of protein concentration
   BCA method was used to determine protein concentration: 0.5 mg/mL standard protein gradient was added to the well of the plate, and PBS was added to make up to 20 µL; an appropriate volume (3 µL) of protein sample was added to the well of the plate, and PBS was added to make up to 20 µL; 200 µL BCA working solution (formulated immediately before use) was added to each well and incubated at 37°C for 30 min; the absorbance at 562 nm was measured, and the protein concentration was calculated from the standard curve and sample volume.
(B) SDS-PAGE gel electrophoresis
   (1) Two cleaned glass plates are aligned and put into a clamp for clamping, and then vertically clamped on a shelf for glue pouring.
   (2) 10% separation gel was prepared by adding TEMED and immediately and uniformly shaking. After that, the glue pouring was carried out and then the ethanol was used to seal. The ethanol on the upper layer of the gel was poured out and dried with absorbent paper when the gel was fully solidified.
   (3) 5% concentrated gel was prepared by adding TEMED and immediately and uniformly shaking. After that, the glue pouring was carried out. The remaining space was filled with concentrated gel and then the comb was inserted into the concentrated gel. After the concentrated gel was solidified, it was gently pulled straight up.
   (4) It was added into the electrophoresis tank and enough electrophoresis solution was added for sample loading. After measuring the protein content of the protein sample, 5×SDS loading buffer was added to 1× final concentration, the mixture was boiled in boiling water for 3 min and mixed well for sample loading with the total protein amount of 35 µg.
   (5) Electrophoresis was carried out at constant pressure of 80 V to run the gel, when the sample entered the lower layer gel, electrophoresis was carried out at constant pressure of 120 V until bromophenol blue reached the bottom of the gel, and then membrane transfer was carried out.
(C) Membrane transfer:
   (1) Gel cutting: The glass plates were pried off, the concentrated gel was scraped off after the small glass plates were removed, and gel cutting was carried out according to the molecular weight of protein and experimental needs with Marker as the control.
   (2) Membrane preparation: A PVDF membrane and filter paper were cut, and the cut PVDF was placed in a 80% methanol solution for activation 30 s.
   (3) Membrane loading: A clamp used in membrane transfer was released to keep a black side (negative electrode) level. A sponge pad was placed on the black side, the transfer buffer was added to soak the sponge pad, the soaked filter paper was placed on the pad, and then the gel, a nitrocellulose membrane, the filter paper and the sponge pad were sequentially stacked in order. Finally, a white plate (positive electrode) was placed and loaded into a membrane transfer tank.
   (4) Membrane transfer: The clamp was placed into the membrane transfer tank to make the black side of the clamp face the black side of the tank and make the white side of the clamp face the red side of the tank. Membrane transfer was carried out at 4°C and constant current of 400 mA.
   (5) The membrane was removed after membrane transfer, and a corner was marked.
(D) Immune response
   (1) Blocking: the membrane was rinsed 3 times in TBST for 5 min each time. After rinsing, the nitrocellulose membrane was added into 5% skimmed milk powder and shaken for 1 h at room temperature.
   (2) Addition of primary antibody: the blocked nitrocellulose membrane was added into a washing tank containing TBST buffer and rinsed 3 times on a shaker for 5 min each time. It was added into a plate with primary antibody and incubated overnight at 4°C.
   (3) Addition of secondary antibody: the primary antibody was recovered, the nitrocellulose membrane in a washing tank containing TBST buffer solution was rinsed 3 times on a shaker for 5 min each time at room temperature, and then the rinsed nitrocellulose membrane was added into a plate with secondary antibody and incubated for 45 min at room temperature in the dark. After the incubation, the nitrocellulose membrane was washed 3 times in a washing tank containing TBST for 5 min each time.
(E) Chemiluminescence
   (1) Reagents A and B were mixed in a small centrifuge tube according to instructions of a luminescence kit and then added onto the nitrocellulose membrane, and a chemiluminescence imager was used for color developing.

Tumor cells maintain their energy requirements for abnormal proliferation and metastasis mainly through changes in metabolism, while changes in metabolic rate are mainly achieved by regulating the expression levels of key genes in the metabolic process. Among them, changes in glycolysis metabolism and glutamine metabolism are particularly important.

It can be seen from Figure 3 that FGF21 injection can significantly inhibit HK2 (hexokinase 2), PKM2 (pyruvate kinase), PFKP (phosphofructokinase), PD (pyruvate dehydrogenase), and GPD (glucose-6-phosphate dehydrogenase) (Figure 3A) that are key proteins for sugar metabolism in tumor, and GLS (glutaminase) (Figure 3B) associated with glutamine metabolism. These proteins are highly expressed in the abnormal proliferation of tumors. A variety of drugs targeting to inhibit these proteins can significantly improve colorectal cancer. The inhibition of the expression of these key proteins by FGF21 further illustrates that FGF21 has a therapeutic effect on colorectal cancer and the anti-tumor effect of FGF21 on colorectal cancer cells is achieved mainly through the inhibition of glycolysis and glutamine metabolism.

The inventor also tried to study the effect of the fusion protein of FGF21 protein as set forth in SEQ ID NO. 2-5 in the treatment of colorectal cancer using the above method. The results show that the FGF21 fusion protein as set forth in any one of SEQ ID NO. 2-5 and/or SEQ ID NO. 11-14 has comparable effects to the FGF21 protein as set forth in SEQ ID NO. 1.

Although the present invention has been disclosed as above in preferred embodiments, it is not intended to limit the present invention. Those skilled in the art can make various changes and modifications without departing from the spirit and scope of the present invention. Therefore, the protection scope of the present invention should be defined by the claims.

## Claims

1. Application of FGF21 protein in the preparation of medicines for preventing, relieving and/or treating colorectal cancer, **characterized in that** the amino acid sequence of the FGF21 protein is as set forth in any one of SEQ ID NO. 1-5 or SEQ ID NO. 11-14.

2. The application according to claim 1, **characterized in that** the effective dose of the FGF21 protein is 0.1-10 mg/kg.

3. A medicine for treating colorectal cancer, **characterized in that** FGF21 protein is used as an active ingredient; and the amino acid sequence of the FGF21 protein is as set forth in any one of SEQ ID NO. 1-5 or SEQ ID NO. 11-14.

4. The medicine according to claim 3, **characterized in that** it further comprises a pharmaceutically acceptable carrier or accessory.

5. The medicine according to claim 4, **characterized in that** the administration route of the medicine comprises oral, intraperitoneal injection, subcutaneous injection, intravenous injection or intramuscular injection.

6. Application of FGF21 gene as a medicine target in the screen of medicines for treating colorectal cancer.

7. The application according to claim 6, **characterized in that** the application is for screening the small molecule activators of FGF21 protein.

8. Application of FGF21 protein in the preparation of inhibitors of sugar metabolism and/or glutamine metabolism in tumor.

9. The application according to claim 8, **characterized in that** the application is for preparing the inhibitors of one or more of hexokinase 2, pyruvate kinase, phosphofructokinase, pyruvate dehydrogenase, and glucose-6-phosphate dehydrogenase that are key proteins for inhibiting the sugar metabolism in tumor.

10. The application according to claim 8, **characterized in that** the application is for preparing an inhibitor of glutaminase.

11. Application of FGF21 protein in the preparation of medicines for preventing, relieving and/or treating cancer.

12. The application according to claim 11, **characterized in that** the application is for preparing medicines for one or more diseases of prostate cancer, breast cancer, esophageal cancer, colorectal adenocarcinoma, cervical cancer, endometrial cancer, ovarian cancer, pancreatic cancer, gallbladder cancer, liver cancer, clear cell kidney cancer, melanoma, and multiple myeloma.
